# EUROPEAN PATENT APPLICATION

(11) **EP 1 178 422 A2**
(43) Date of publication of application: **06.02.2002**
(21) Application number: 01202934.4
(22) Date of filing: 02.08.2001
(51) Int. Cl.: G06F 17/60

(54) **Registration and ordering system**

(30) Priority: 04.08.2000 US 222807 P
(71) Applicant: SmithKline Beecham Corporation, Philadelphia, PA 19103 (US)
(72) Inventor: Hetzel, Frank, Collegeville, Pennsylvania 19426 (US); Kirsch, Richard, Collegeville, Pennsylvania 19426 (US); Schindler, Edward, Collegeville, Pennsylvania 19426 (US); Walsh, Terrence, Collegeville, Pennsylvania 19426 (US)
(74) Representative: Giddings, Peter John

(57) **Abstract**

This invention relates to a process and system for managing blinded studies such as medical clinical trials. It is made up of three components a supply tracing approval module, a module for randomizing supplies and an interactive voice or online response system for requesting materials.

## Description

This invention relates to a process and system for managing blinded studies such as medical clinical trials. It is made up of three components, a supply tracking and approval module, a module for randomizing supplies, and an interactive voice or online response system for requesting materials.

### Background

Drugs and vaccines must be tested extensively to insure safety and efficacy before they can be released for general sale to the public for treating or preventing diseases in man or animal. These studies proceed through several phases, usually identified as Phase I, Phase II and Phase IIIa and Phase IIIb/IV clinical trials. In each of these phases medical research groups must be identified and approved prior to participating in a study. Study protocols must be approved. Patients need to be recruited, screened and enrolled in the study. The study must be blinded so neither any of the parties know who is receiving active or placebo treatment until after the study is completed. Sample order processing must reflect this need for anonymity.

The number of patients in each study must be tracked along with the total number of patients in all of the study centers. Progress and completion data must be accumulated and tracked as well. Large-scale clinical studies can involve tens of different centers in several countries, each at a different stage of enrollment and completion. Since blinded, randomized treatment must be followed to determine efficacy, drug supply can present a challenge especially where large numbers of patients are enrolled at geographically remote study sites.

Each new drug or vaccine to be tested involves a unique set of test parameters and methods that usually must be tracked in a unique way. So the computer code written for managing the algorithm in one study does not travel to the next study under current practices; it must be rewritten extensively before it can be rolled out to manage a new study.

There is a need for a method and system to provide centralized control of clinical study supplies which provided real-time information about supply usage and active centers and patient enrollment by center. This system needs to be able to monitor enrollment to insure it is completed on time, that the number of enrolled patients in a multi-site study do not exceed the total number called for in the study protocol and that supplies are shipped to the proper site for each patient. It also needs to be able to track patient registration and randomization and have the capability of immediate contact with all sites, i.e., a broadcast technology. In addition, the system must be able to record and provide information on approved and terminated site to insure compliance with regulatory provisions and provide notification about non-compliant sites if compliance becomes an issue. Monitoring site documentation is also a factor that should be part and parcel of the system.

This invention addresses these needs and several inefficiencies inherent in historical methods used to manage clinical trials. It provides a means for tracking efficiently the status and activities of multiple sites; reduces the need for raw materials, storage and handling of trial samples; and is portable. Thus it can be used to manage the study of many drugs and vaccines without having to re-program the code. While this invention has origins in the context of clinical trial work, it can be used in any environment where randomized, blinded testing is carried out.

### Summary of the Invention

In a first aspect, this invention relates to a registration and sample ordering system for blinded, randomized testing of samples of a product at one or more sites, the system comprising:
i) a remotely accessible electronic database programmed to:
   a) run real-time or batch data processes,
   b) process at least two call flows,
   c) receive and process test subject registration data,
   d) randomize subjects, and
   e) lock out new subjects when study numbers are fulfilled; and
ii) an interactive electronic ordering and tracking system for supplying samples to one or more sites, said ordering system having an electronic randomizing operation for randomizing and individualizing samples based on prior subject randomization.

In a second aspect this invention relates to a method for carrying out blinded, randomized testing of samples of a product at one or more sites, the method comprising:
i) remotely accessing an electronic database programmed to:
   a) run real-time or batch data processes,
   b) process at least two call flows,
   c) receive and process test subject registration data,
   d) randomize subjects, and
   e) lock out new subjects when study numbers are fulfilled; and
ii) interactively electronically ordering and tracking samples for one or more sites, said sample ordering system having an electronic randomizing operation for randomizing and individualizing samples based on prior subject randomization.

### Description of the Figures

Fig. 1 is an overview of a registration and ordering system in three parts.

Fig. 2 is a flow diagram of the operational parts of a system.

Fig. 3 illustrates the difference in materials requirements between a block randomization system and a virtual randomization system.

Fig. 4 is a flow diagram of an electronic ordering and supply system.

Fig 5 is a top-level screenshot of a set of Lotus Notes folders and views.

Fig 6 is a top-level screenshot of an exemplary folder used to identify and defined a distribution center.

Fig 7 is a screenshot of exemplary fields used to identify and defined a distribution center.

Fig 8 is a screenshot of a top-level listing of a clinical protocol.

Fig 9 is a screenshot of a second-level listing of a clinical protocol.

Fig 10 is a screenshot of an exemplary kit definition.

Fig 11 is a screenshot of a set of exemplary test kit identifiers.

Fig 12 is a screenshot of an exemplary test kit identified as awaiting approval.

Fig 13 is a screenshot of an exemplary listing of information for a clinical investigation center.

Fig 14 is a screenshot of an exemplary listing of centers by country.

Fig 15 is a screenshot of a clinical supplies order form.

Fig 16 is a screenshot of a shipping operation.

### Detailed Description of the Invention

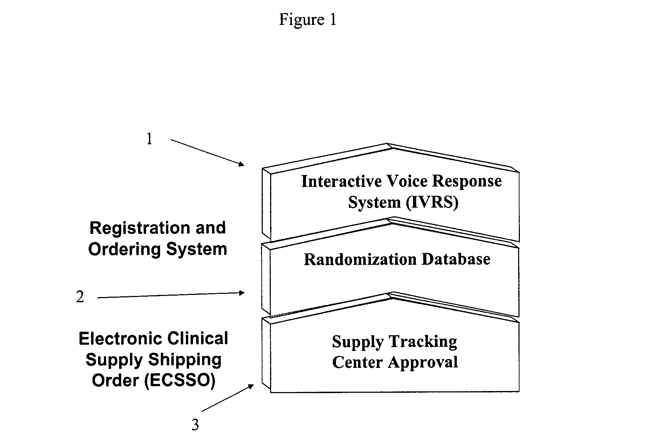

In its broadest utility, this system and method can be used in managing the testing of product samples using either open-label or blinded, randomized testing techniques. Figure 1 illustrates the overall system. It has two functions which are illustrated by three blocks: i) an interactive registration and ordering system comprising a database (2) for randomizing patients and study samples and a data capture system herein illustrated by an interactive voice response system (1); and ii) an electronic tracking center (3) for receiving and shipping randomized test samples based on information provided by data base (1).

The core of the operation is a relational or quasi-relational data base programmed to carry out the two main functions of the operation and the several subordinate routines supporting those two functions. This database will be accessed using client-server operating systems, terminal emulation, a web browser or similar internet-type communication device. While these devices and systems currently are primarily keyboard data-entry based, it is contemplated that voice recognition technology will soon become sufficiently advanced to permit voice and/or audio command and control of the entire system. In fact some of the activities of the second function, that of ordering and tracking sample, can be accomplished using keyboard input or voice, or more accurately speech, recognition technology; voice recognition technology being a preferred means for placing orders. Output to printers and facsimile machine and other telecommunication devices will also be integrated into the system and linked to the database software and hardware.

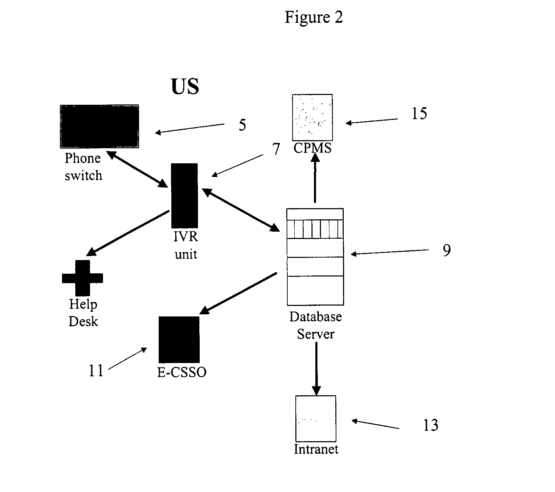

Figure 2 is an overview of the hardware and operational units of the system described herein. In this figure, the database runs on database server (1). Preferably server (9) will be housed in a secure environment and the operating system and database software running on it will have security controls built into it. This server (9) is accessible phone using the voice recognition system (7). It is labeled as an IVR Unit in figure 2. IVR is the acronym for interactive voice recognition. Server (9) can also be access from a personal computer connected in via a network (15) or over an Intranet or extranet (13). These latter connections provide the means for inputting data, viewing records and generating reports, as illustrated by the two-way arrows between the server and the voice recognition units. The server is also linked to electronic supply and shipping system (11), identified as E-CSSO.

The system exemplified here has been designed to run on off-the-shelf hardware and software. Any computational device capable of acting as a server can be used on the backend. Exemplary machines are Intel-based computers or Sun Microsystems workstations using a UNIX or Windows NT or Linux operating system. Any personal computer (PC or Macintosh) can be used as the client, or some other device capable of linking up to the server such as the likes of a web TV device, a personal data assistant (PDA), a web-enable cell phone, or the like.

Any relational or semi-relational data base can be used, provided it has the capacity to handle the number of records in a given study, and is capable of being programmed to handle the multiple data field links needed to perform fully the functions of the algorithm described here. Databases such as Access, FileMaker Pro, Lotus Notes, Oracle, Sybase, Titanium, and the like can be used. It is preferable to use a high-capacity database such as Lotus Notes, Oracle or Sybase to avoid undertaking as study, only to find the number of files exceeds the capacity of the smaller-capacity database like Access or FileMaker Pro.

In addition this system will have integrated into it an interactive phone response system (7). Facsimile capability is also built into the system for distributing certain types of data.

"Voice recognition" and "speech recognition" is used interchangeably herein. Speech recognition is the field of computer science that deals with designing computer systems that can recognize spoken words, as compared with recognizing a pattern of sounds and associating it with a unique source, i.e., personal identification-type technologies. Note that speech recognition implies only that the computer can take dictation, not that it *understands* what is being said. Comprehending human languages falls under a different field of computer science called *natural language processing.* A number of speech recognition systems are available on the market. The most powerful can recognize thousands of words. However, they generally require an extended training session during which the computer system becomes accustomed to a particular voice and accent. Such systems are said to be *speaker dependent.* Many systems also require that the speaker speak slowly and distinctly and separate each word with a short pause. These systems are called *discrete speech* systems. Recently, great strides have been made in *continuous speech* systems -- voice recognition systems that allow you to speak naturally. There are now several continuous-speech systems available for personal computers. Because of their limitations and high cost, speech recognition systems have traditionally been used only in a few specialized situations. For example, such systems are useful in instances when the user is unable to use a keyboard to enter data because his or her hands are occupied or disabled. Instead of typing commands, the user can simply speak into a headset. Increasingly, however, as the cost decreases and performance improves, speech recognition systems are entering the mainstream and are being used as an alternative to keyboards. In this invention, the latter type of speech recognition is the preferred system.

Developments in speech recognition systems are progressing rapidly and globally. Information on these developments and currently available systems available via the Internet. Examples of current speech recognition software are Lenrout & Hauspie's VoiceXpress suite of software including VoiceXpress Mobile; Dragon Systems, Inc.s' Dragon NaturallySpeaking suite of software; and IBM's ViaVoice™ Millenium for PCs and Via Voice for Macintoshes. In addition, speech recognition microphones are available from Plantronics, Buddy Microphones, Parrott, Shure, Emkay, Telex, Andrea and Philips. Speech recognition recorders are made by Olympus, Voice IT, Sony Sound Cards, and Norcom, to name some current manufacturers. Information about these companies and their products can be found on the Internet. Custom voice recognition and interactive phone response systems can also developed; the detailed system described herein uses a custom-made voice recognition system.

### System Description

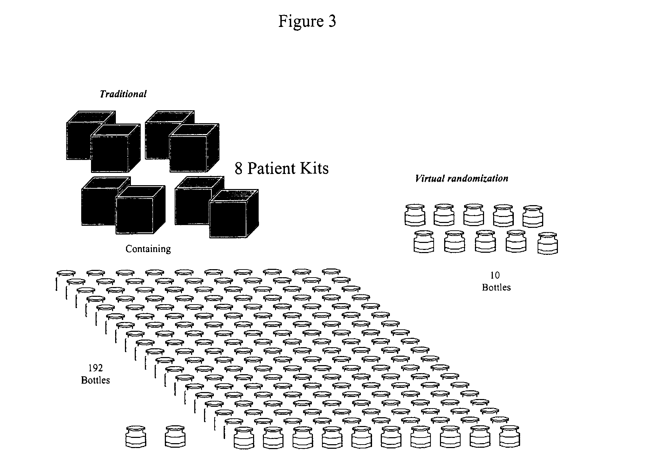

A core component of this operation is the virtual randomization system and the electronic supply and shipping operation integrated with it. It reduces significantly the number of samples, and consequently the amount of material, that are required in order to carry out a randomized product study. Figure 3 illustrate how this virtual randomization system compares with that of a traditional block-randomization system. To supply the proper samples for 8 patient kits requires 192 sample bottles if done by the traditional block randomization system, but just 10 bottles of product sample are needed using the virtual randomization system described herein. And equally important is the fact that this virtual randomization only requires a change in study-specific parameters in the database, not in the flows, for a new study or study protocol. This saves significant re-programming effort; traditionally each new study protocol required re-programming the database to reflect changed and/or new parameters and steps needed to carry out the new study or new study protocol.

This virtual system is based on six different fixed flows which cover all flows one would use in a product trial, namely: registration, open label, run-in, double blind, stratified, and titration. Patient registrations can be accepted directly into the database (9). It also randomizes patients and is set to lock out additional enrollees when enrollment targets are reached. Oracle is the preferred database for this operation.

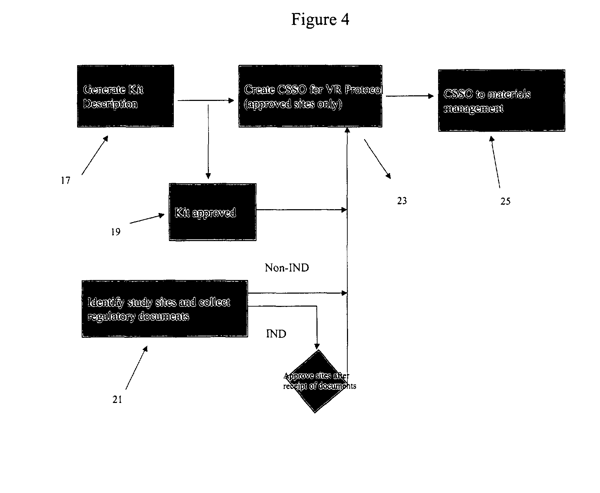

The electronic clinical supply shipping order system (ECSSO) has been created to ensure sites are approved for use, cut off non-compliant sites, monitor site documentation, and ensure appropriate filings have been made in all participating countries. Figure 4 illustrates the ECSSO process.

The first thing that is set up is the kit description (17). This description is prepared based on the parameters set out in a particular study protocol, approved (19) and entered into database (9) in figure 2. It describes the identity of the samples that need to be sent to each study participant and identifies the ancillary materials that may be needed for a particular study or sub-routine in a study. For example, when a drug clinical trial is set up, a protocol for testing the drug is agreed. This protocol defines the dosage, form of the drug delivery system, e.g., an oral tablet, when and how frequently the tablet is to be taken and for how many days, and possibly a diary for recording reactions or events which may be needed to identify efficacy or track adverse reactions. In this scenario, the kit would contain at minimum a bottle of tablets with instructions for taking the tablets and perhaps a diary of some kind for recording compliance with the dosing regimen.

One the kit description is prepared and recorded, it becomes the basis for programming the clinical supply shipping order system and integrating it with voice recognition technology (23). This data also resides on server (9). Study sites and study documentation (21) entered onto the database on server (9). This work can be done in parallel with setting up the kit description and creating the CSSO and IVR systems. If the study is one that is characterized as an investigatory new drug (IND) study, the sites must be approved before they can be linked to the CSSO/IVR operation (23). If the study is a non-IND study, then the function of box (21) can be linked to the CSSO/IVR function (23) within having to go through a second approval step. The CSSO code is then provided to the warehouse or materials management operation (25). That part of the operation receives orders originating through the IVR and assembles and ships kits based on virtual randomization profile created on the database on server (9).

A more detailed description of the invention follows.

### Detailed Description of ECSSO

A specific embodiment of this invention is based on a networked Lotus Notes (version 4.51) application. It contains Word 97 templates for generating shipping labels and shipping documents. This database contains the relevant and necessary information to ensure efficient clinical supply drug distribution in compliance with good manufacturing practices, good clinical practices and country-specific medication related regulations. This application can be viewed by all Lotus Notes users who are authorized to access it within certain company-designated domains, and also by third parties who have replicated the database to their local servers. Document creation and modification privileges are given only to approved staff and third parties.

Author access is granted to the database as needed by one of the system administrators. There are two system administrators, primary and backup. In order to grant author access in the commercial domain there is also a commercial custodian. Only authorized administrators can make additions, deletions or modifications to the Lotus Notes access control list. The system administrators also have access to all functionality available within the database and are the only staff with edit and delete capability.

The electronic clinical supply application (ESCCO) has eight distinct operations. These include:
1. Protocol and supply distribution management.
2. Entering investigational sites.
3. pproval of investigational sites.
4. Approval of the investigational site.
5. Entering the clinical drug supply description.
6. Approval of the drug product.
7. Ordering the clinical drug supplies.
8. Shipping of clinical supplies.

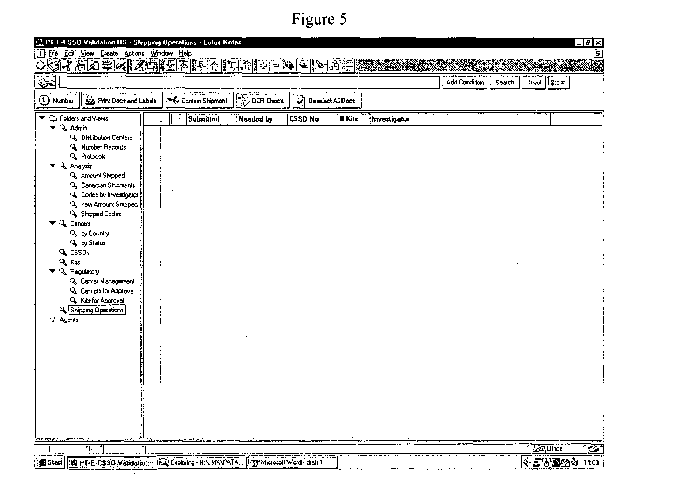

The database fields and functional capabilities assigned to the different roles in this application are detailed below. Current SOP has each of these 8 operations being managed by a different person or group, although one person or group could be responsible for two or more of these operations. The top-level view of this LN database is shown in figure 5.

### Distribution Center and Protocol Setup

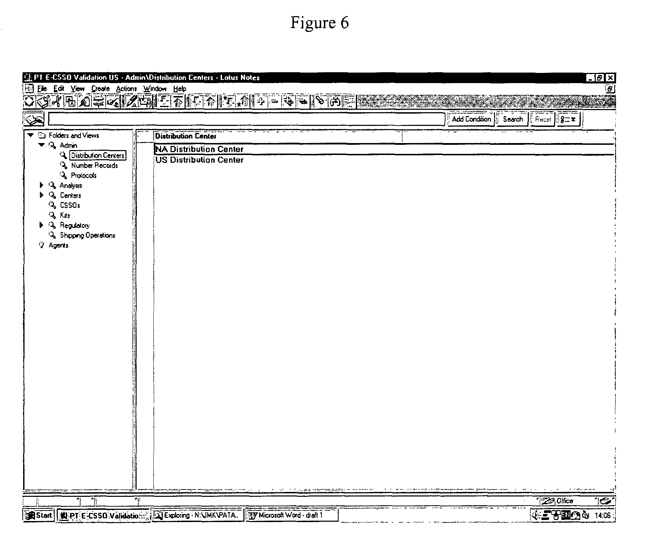

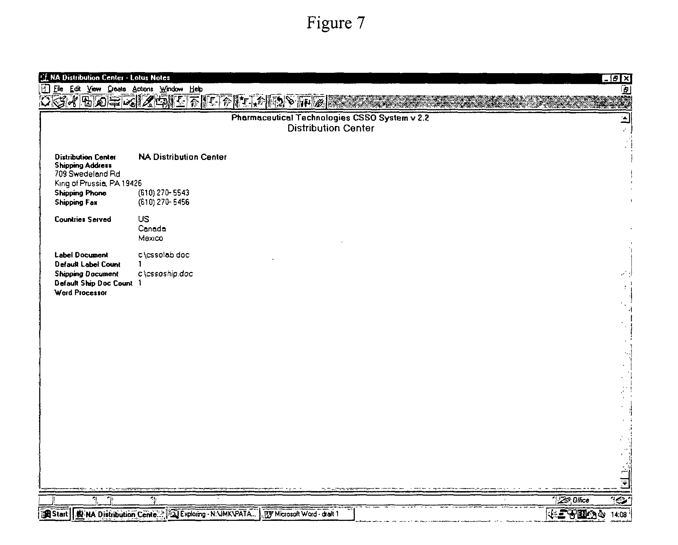

System administrators, in addition to overall database management, are responsible for initiating the process through the establishment of supply distribution centers (figures 6 and 7). The *Distribution Centers* view is shown in figure 6. This view lists the sites that have been established as supply depots that may be used to supply clinical investigators in Europe and North America. In order to establish a new distribution center, the system administrator selects *Create...Distribution Center* from the top-level menu and enters the mailing address telephone and fax numbers for the distribution site in the relevant fields of the distribution center form (figure 7). The countries that an established distribution site can supply are created using the pull down menu shown in figure 6. Distribution centers are allowed to distribute supplies only to countries designated on the form. Additional countries may be added, as appropriate, by one of the system administrators. Administrators will add countries to a distribution site in response to instructions by a designated management function. Also located on this form, is the path for accessing the locally customized, label and shipping document templates, as well as, the printing defaults. Templates/documents are stored locally for access by shipping staff. These templates will be provided to the local Notes administrator and must be installed by the local computing support staff with help of one of the database administrators. As shown in figure 7, the current default is set to print 1 shipping label and 1 shipping document. The printing defaults can be customized based on local practice, and can be changed by the local Notes administrator in response to need. Similarly, the default values can be overridden by shipping staff where appropriate during shipping operations.

### Handling Protocols

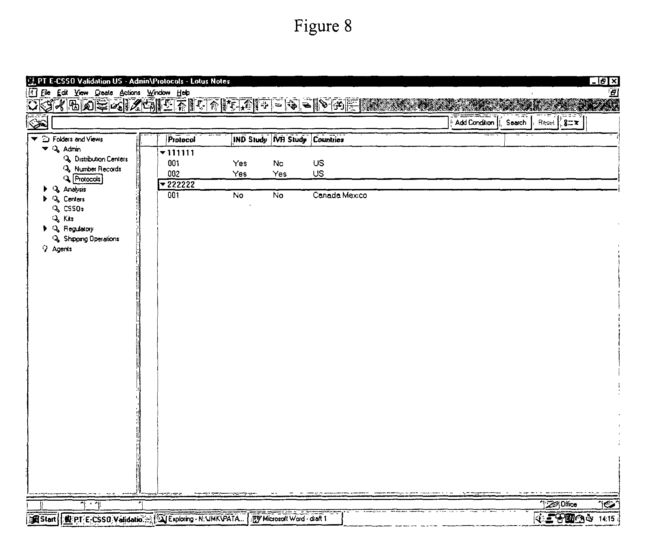

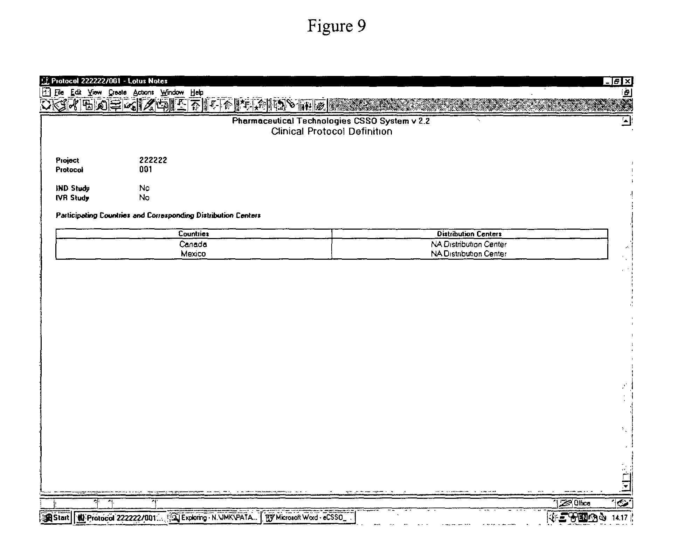

Protocol specific information is entered by the designated study manager The *Protocol* view is shown in figure 8. In order to configure a new protocol in the database, the manager selects *Create...Protocol* from the top-level menu, which opens the form shown in figure 9. Using the functionalities associated with the various electronic buttons, participating countries and associated distribution sites are entered by a designated representative. Additional participating countries can be added to the list, the distribution center supplying a specific country can be changed or a country can be removed from the participating list. Ultimately, the person or group responsible for packaging is responsible for insuring that the country list corresponds with the agreed country list.

Of critical importance on the Protocol form is the identification of protocols to be conducted in compliance with investigatory new drug (IND) guidelines. For purposes of this database, investigatory new drug studies are operationally defined as those clinical protocols that require study site approval prior to center initiation. Under current standard operating procedures and policies, all studies with US participation will require site approval prior to supply shipment and site initiation. All studies with US participation will be designated as IND studies. Once selections are finalized, select Escape and the standard Lotus Notes procedures are evoked.

### Entering and Approving Kit Descriptions

Following creation of a protocol document, the description(s) of the packaged study medication to be supplied in the protocol are entered into the database by a designated party. A kit is created as follows:

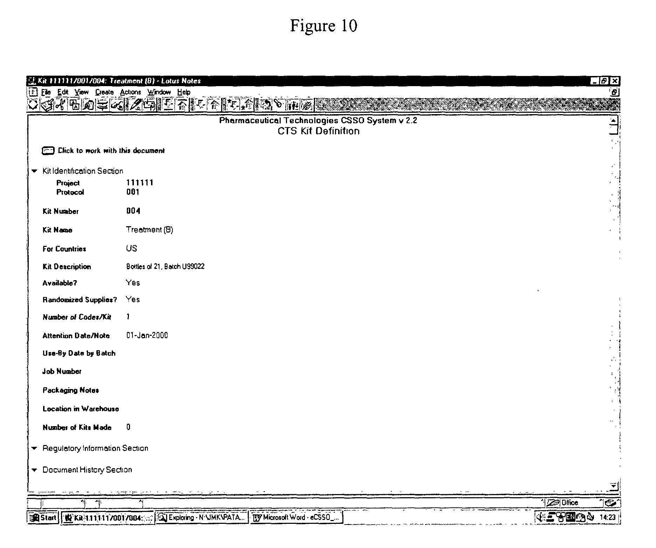

Entry is initiated via selecting *Create*...*Kit* from the top-level menu options. This accesses the Kit Definition Form. A completed sample is shown in figure 10. Project and protocol are selected from the available menu options on the form. Kit descriptions cannot be entered until the protocol is entered as described above. Following selection of the protocol, designated managers must enter a three digit Kit number for proper supply identification with the first Kit assigned to each individual specific protocol starting with 001. Similarly, the kit name (e.g. Run In, Treatment, etc.), the countries that these supplies are intended to be used in and a detailed description of the packaged product must be entered in the appropriate fields. Information as to whether these are randomized supplies the number of patients supplied per kit, use by date by batch, must also be entered in the appropriate fields. For inventory purposes, the exact warehouse location and the total number of kits created may also be captured on the appropriate section of the form. Operationally, all supplies that have been issued a specific, unique container/randomization number should be defined in the database as randomized. Supplies that have not been assigned specific container numbers, such as open label extension supplies, as well as, those intended for third party blinding should not be considered randomized. To simplify operations, there is also a field on the form called "Available?" When the radio button associated with this field is selected as NO, a minus sign (-) is entered into the *Kit* view next to the Kit name. This symbol indicates that the supplies from this packaging job are not yet available for shipment or that they have been exhausted and there are no additional supplies from this packaging available for this protocol. Moreover, when the NO button is selected, these supplies will no longer appear in the pull down Supply Selection submenu of the CSSO form (see figure 10).

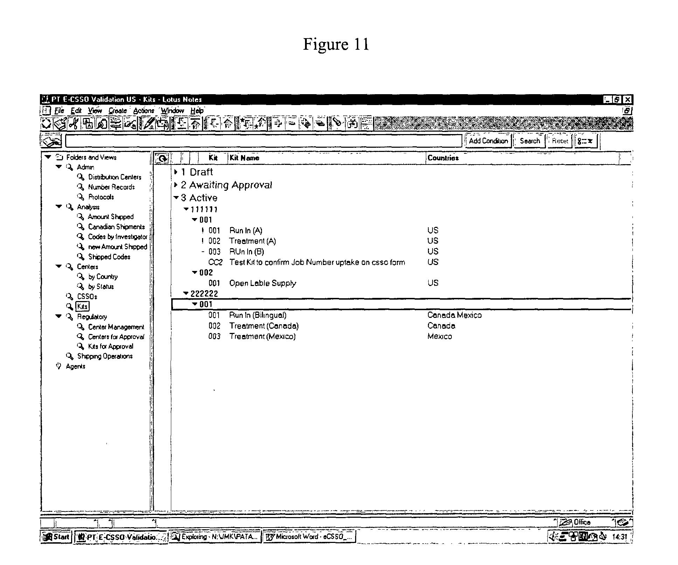

There is also a field on this form for establishing an Attention Date and a descriptive note associated with this date. This date is used as a flag by the designated packaging representative to indicate that something must be checked associated with the supplies. When the attention date is passed, an exclamation point (!) is entered in front of the Kit description in the Kit view (see figure 11). Double clicking on the Kit description will open the associated document and the note will become accessible. An exclamation point does not necessarily indicate that the use by date has expired.

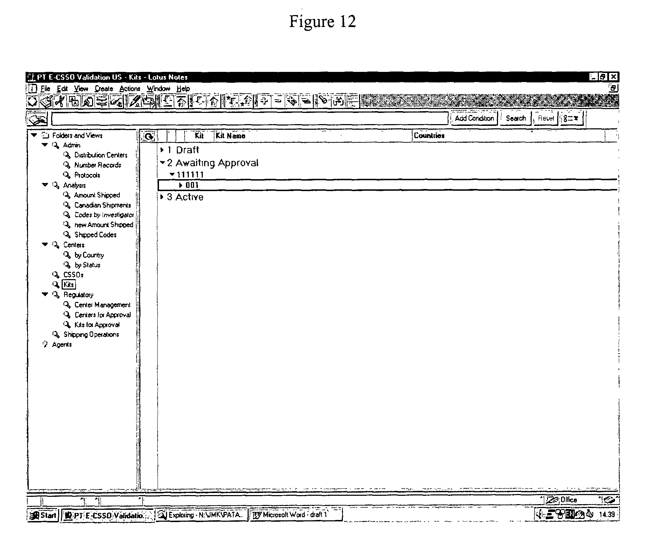

Once all of the necessary information is entered on the form, the Kit Definition Form is submitted to the appropriate staff for approval. This is done by selecting the *Submit this kit for approval* button. Selecting this button does initiates two processes. First, the Kit Definition Form changes state in the workflow into Awaiting Regulatory Approval view in the *Kits* folder (figure 12) and also into the Kits for Approval view of the Regulatory folder. Second, this application also prepares an e-mail message to be sent to the relevant staff indicating that approval of the packaged product is requested. When the staff approve the kit for use, the Kit Definition Form is removed from the Kits for Approval view and also changes state from Awaiting Approval to *Active* in the *Kits* views (see figure 11). Operationally, approval indicates that the necessary documentation was submitted to the FDA has been given to the appropriate department for subsequent local country filing. Once active, the kit can be accessed for ECSSO preparation. Unapproved kits cannot be used to prepare CSSO. In parallel with approval in the database, an e-mail is sent to the packaging manager(s) indicating that the kit has been approved for use.

### Entering and Approving Center Information

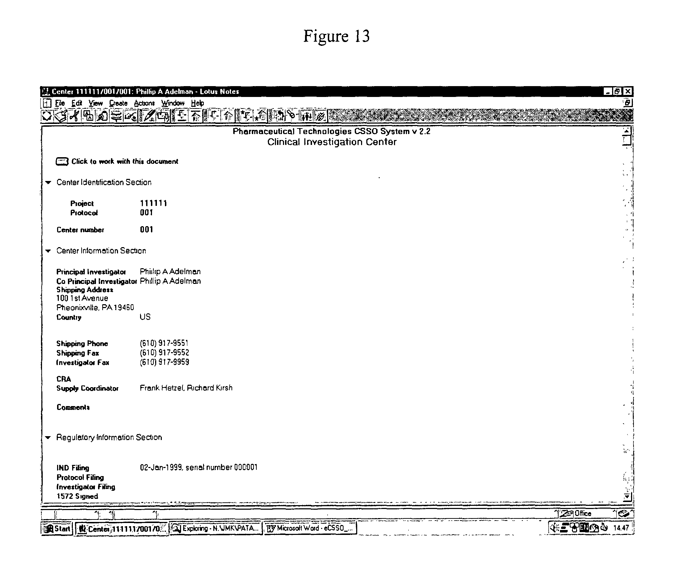

Once a protocol is entered into the database, the clinical staff is able to begin entering potential investigators/study sites into the database. This process is initiated by selection of the *Create...Center* top level menu. This opens the Clinical Investigation Center form (see figure 13 for completed form). On this form, the project and protocol are set by use of the menu lists under *the Set Project and Protocol* button. Once selected, the clinical representative enters the center number and the relevant center information, including Principal Investigator (PI), Subinvestigators, Country and actual Shipping Address. This address, for IND studies, should be the address found in box 3 of form 1572.

In the current structure of the database, it is important to note that the ECSSO pulls the name and address information for the shipping document and label directly from the center document. As such, the mailing address must be correct in order for the supplies to reach the correct investigator. In the current version of the database the sub investigator is operationally defined as the actual person conducting the investigation at the study site. As such, the "ship to:" name will be the person(s) listed in the Subinvestigator field. If the PI needs to be identified on the shipping document, he/she must be identified in the Subinvestigator field. The designated representative should also enter, if possible, in the Study Coordinator or research associate fields, all of the ancillary staff that needs to know when the center is approved for use. If this information is entered an e-mail will be generated automatically when the center is approved for use by staff (IND studies only).

If the information is incomplete or believed to be immature and not ready to be submitted for regulatory approval, the clinical representative has the option of pressing the button labeled *Save with no Workflow*. This places the document in Draft state. When the document is completed, for IND protocols, the button labeled *Submit this Center for Approval* is selected. This button changes the state from Draft to Awaiting Regulatory Approval in the Status view of the Centers folder

Similarly, the status changes in the Country view of the Centers folder and the center appear in the Centers for Approval view of the Regulatory folder. Concurrent with this change in state, a message box appears interrogating, *Has an investigator agreement been received?* If not received is indicated, the CIF also appears in the Awaiting Investigator Approval view in the Center folder. In this situation occurs, the clinical person must first select the button labeled *Indicate Investigator Agreement has been Received* in order to allow supply shipment. The indication that the investigator agreement has been received is required independent of regulatory approval. Both conditions are required for a center to be activated in the system to receive clinical supplies.

Once a center form is submitted to Regulatory Affairs and the RA associate has confirmed the suitability of the submitted site documentation, they can approve the site for clinical use, by selecting the *Approve this center* button on the form. It is recommended that the RA associate also enter, at a minimum, the institutional review board's (IRM) expiration date and the investigator's license expiration date in the relevant field. Although not currently mandatory for approval, use of these fields facilitates tracking of site document expirations by designated parties. If the IRB expiration and license expiration dates are entered, the database can also be used to track these expiration dates. An exclamation point is entered next to the investigator name in the *Center Management* view of the *Regulatory* folder if one of the expiration dates has passed. Regulatory Affairs also has the exclusive authority to remove approval of sites to receive supplies due to any perceived regulatory issue. This is most efficiently handled in the Center Management view within the folder. In order to remove approvals, select the centers of interest with a check and press the Remove Approval button at the top of the form. If regulatory approval is removed, the Center document changes state in the workflow from Approved to Awaiting Regulatory Approval. When RA evokes this authority, a mail message is automatically generated and sent to the study coordinator indicating that regulatory approval has been removed and additional supplies cannot be shipped until the information is updated. When the regulatory information is updated, RA can reapprove the site for clinical use. Upon re-approval, the standard mail message appears addressed to those in the study coordinator field of the clinical information section, indicating that the center has been re-approved for use. The study coordinator should forward this message to anyone else that requiring notification.

Following regulatory approval, it is often necessary to change or update information that was entered by clinical, due to changes in personnel coverage or new addresses and telephone numbers, etc. This can done by clinical selecting the button labeled... *Unlock Center Information.* This places the CIF back into Draft mode and allows information to be added in the clinical database fields. Only modification, to the CRA field, or telephone numbers by clinical staff does not require re-approval by RA to ensure that the information complies with all regulatory requirements. The final button *Deactivate this center* irreversibly insures that the supplies cannot be sent to the selected investigator and places the investigator in an inactive folder. Once in the inactive folder, the investigator can only be removed through manual intervention by one of the database administrators.

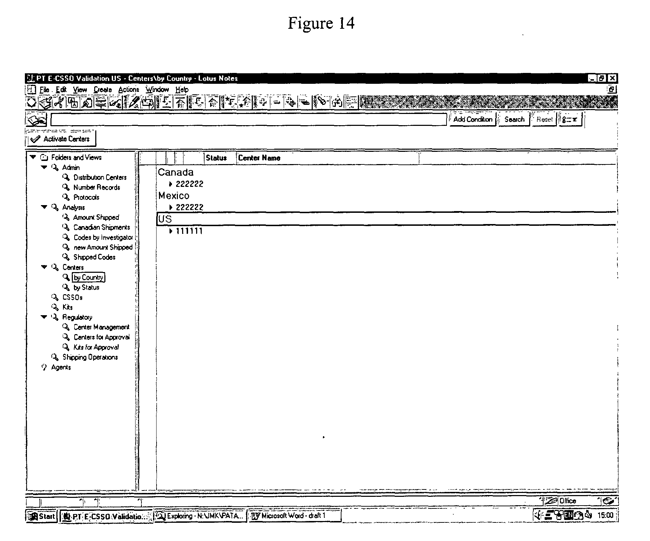

For non-IND studies, the regulatory approval step is bypassed and clinical can approve the sites directly. Using the country view in the center folder, it is also possible to activate multiple centers simultaneously. This was designed for situations, such as in France, where receiving Ethics Approval for one center opens the opportunity to activate all of the sites. In this situation one should check all of the sites intended for activation and press the button at the top of the view stating *Activate Centers* (figure 14).

### Generating an ECSSO

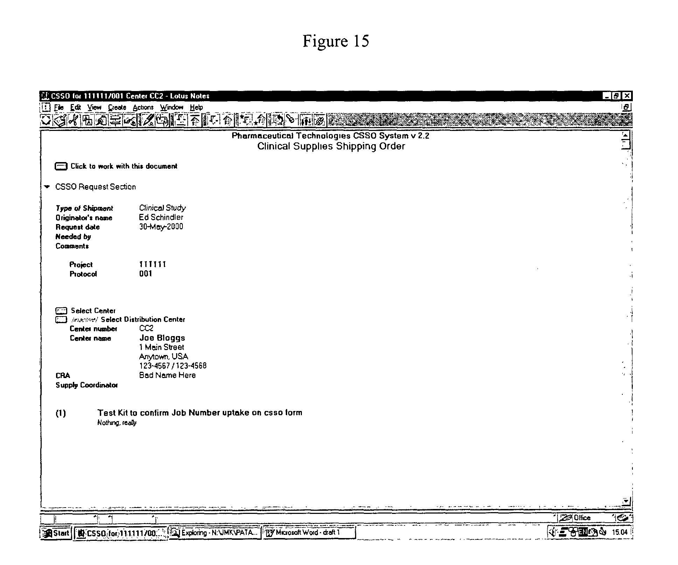

The clinical functionality also allows preparation of an ECSSO request. This option is selected by using *Create*...*CSSO* from the main menu. The process is initiated by selecting the project and protocol from the drop down menus associated with the *Select Project and Protocol* button of interest on the Clinical Supply Shipping Order form (figure 15).

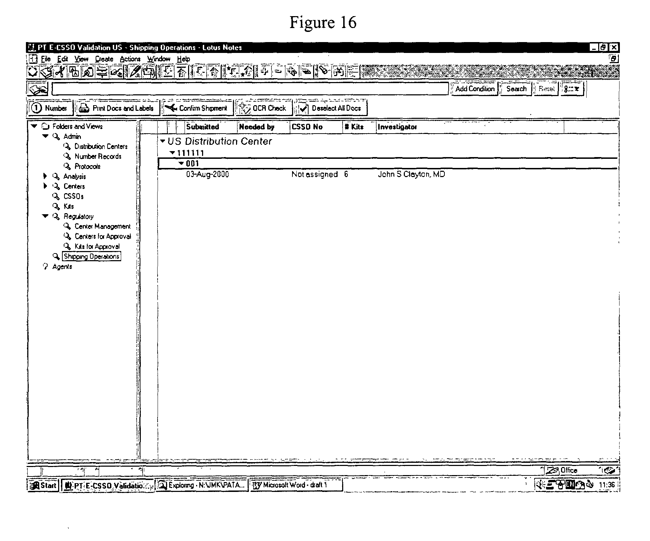

Following selection of the correct study, the *Select Center* button opens a drop down menu containing only centers that are approved for use by RA (IND protocols) or clinical staff (non-IND protocols). The Active Center menu uses the name that appears in the sub investigator field. Following selection of a center from the menu, the Kits required to supply the selected investigator with appropriate medication is selected from the pre-approved packaged products. For studies supplied with randomized double blind medication, the system requires entry of the actual randomization numbers or container numbers to be sent to an investigation site. These numbers must be listed, comma delimited or hyphenated to define a range (e.g. 2,5,6,7-22), in the *Codes* field provided. An ECSSO cannot be generated for randomized supplies without entering the specific codes required. Moreover, as described above, the number of codes requested, must match the number of patient's supplies in each shipping container. For example, if four codes are requested, and a shipping container has supplies for four patients, the number of shipping containers requested must equal one. Once completed, the button labeled *Submit to Distribution Center* is selected and the ECSSO is automatically transferred to the *Shipping Operations* folder and found under the relevant distribution center heading (figure 16).

The ECSSO also appears in the submitted view of the CSSO folder. There is also a field on the CSSO request form for inputting information on the Date Needed. This field is only required if there is a specific due date for the requested supply delivery. This can be used for emergency shipments or to indicate the need for a weekend delivery. In the event of an emergency shipment, be sure to follow up the CSSO request with electronic mail to the shipping operation staff indicating the nature of the emergency and availability of staff to receive the supplies on the requested date.

### Processing an ECSSO and Shipping Supplies

In order to process supply shipments, shipping operations staff initiates action by selecting the days requests with a check mark and pressing the *Number* button in the shipping operations view see figure 16. This action enters a distribution center-specific, unique, sequential number which is used for tracking purposes, on the shipping document. This number also serves as a "return receipt" to indicate that the order will be acted upon by the shipping group within the next 24 hours. Once numbered, the shipping operations staff can select and print the label and shipping documents in WORD 97, using the *Labels* and *Ship Docs* buttons at the top of the view followed by standard Microsoft print commands. Conversely, if there is any confusion or technical issues with the request, shipping operations staff have the option of sending the ECSSO back to the draft state, with notification sent via E-mail, to the originator, that corrections are required. In order to return the CSSO shipping operations staff must open the actual CSSO and select the *Return to Clinical* button. The originator may either modify or correct the returned ECSSO or, if more convenient, generate a new ECSSO. Once the ECSSO is processed, packaged and dispatched, shipping operations issues an electronic confirmation of shipment via E-mail by depressing the *Confirm Shipment* button at the top of the view. This message is sent to those individuals listed in either the CRA or the Supply Coordinator field in the ECSSO document. This action also transfers the processed ECSSO from the *Awaiting Shipment* State in the CSSO view, and automatically enters the current date in the database. A signed, hardcopy record of the shipment is retained with the official packaging documentation.

The foregoing descriptions and discussion of its operation is provided to illustrate how the invention works. These descriptions and discussion are not to be taken as limiting the invention; rather reference is made to the claims herein for what is reserved to the inventors hereunder.

## Claims

1. A registration and sample ordering system for the blinded, randomized testing of samples of a product at one or more sites, the system comprising:
i) a remotely accessible electronic database programmed to:
a) run real-time or batch data processes,
b) process at least two call flows,
c) receive and process test subject registration data,
d) randomize subjects, and
e) determine when study numbers are fulfilled and thereafter lock out additional subjects;
ii) said database being linked to an interactive electronic ordering and tracking system for supplying samples to one or more sites, said ordering system having an electronic randomizing operation for randomizing and individualizing samples based on prior subject randomization.

2. A computer-based method for carrying out blinded, randomized testing of samples of a product at one or more sites, the method comprising:
i) remotely accessing an electronic database programmed to:
a) run real-time or batch processing of data,
b) process at least two call flows,
c) receive and process test subject registration data,
d) randomize subjects, and
e) determine when study numbers are fulfilled and thereafter locking out additional subjects; and
ii) linking said database interactively to a second electronic system programmed to accept orders for and track distribution of samples to be used at one or more sites, said sample ordering system having an electronic randomizing operation for randomizing and individualizing samples based on prior subject randomization.

3. A method for registrating and ordering of samples for blinded, randomized testing of samples of a product at one or more sites, the method comprising:
i) remotely accessing an electronic database programmed to:
a) run real-time or batch processing of data,
b) process at least two call flows,
c) receive and process study subject registration data,
d) randomize subjects, and
e) determining when study numbers are fulfilled and thereafter lock out additional subjects.

4. The method of claim 3 wherein said database is programmed to communicate with and provide instructions to an interactive electronic ordering and tracking system for supplying samples to one or more sites, said ordering system having an electronic randomizing operation for randomizing and individualizing samples based on prior subject randomization.

5. A registration and sample ordering system for blinded, randomized testing of samples of a product at one or more sites, the system comprising:
i) a remotely accessible electronic database programmed to:
a) run real-time or batch data processes,
b) process least two call flows,
c) receive and process test subject registration data,
d) randomize subjects, and
e) lock out new subjects when study numbers are fulfilled; and
ii) an interactive electronic ordering and tracking system for supplying samples to one or more sites, said ordering system having an electronic randomizing operation for randomizing and individualizing samples based on prior subject randomization.

6. A machine-readable medium which has written thereto code for controlling a computational device in a manner which causes it to carry out a method for blinded, randomized testing of samples of a product at one or more sites, wherein the code comprises:
i) a database with remote access capability and programmed to:
a) run real-time or batch processing of data on said device or controlling said processing of data on a remote device,
b) handle at least two call flows,
c) receive and process directly or remotely test subject registration data,
d) randomize subjects, and
e) determine when study numbers are fulfilled and thereafter lock out additional subjects;

7. The medium of claim 6 wherein said database is interactively linked electronically to a system for ordering and tracking sample supplies for one or more sites, said sample ordering system having an electronic randomizing operation for randomizing and individualizing samples based on prior subject randomization.

8. An electronic system for ordering and tracking sample supplies for one or more sites at which are being carried out a blinded, randomized testing of samples of a product, said sample ordering system having an electronic randomizing operation for randomizing and individualizing samples based on prior subject randomization and, optionally, capable of being linked interactively to a second database or system according to claim 6.
